# EUROPÄISCHE PATENTSCHRIFT

(11) **EP 1 534 151 B1**
(45) Veröffentlichungstag und Bekanntmachung des Hinweises auf die Patenterteilung: **25.05.2011**
(21) Anmeldenummer: 03747931.8
(22) Anmeldetag: 26.08.2003
(51) Int. Cl.: A61B 17/28, B25B 7/06

(54) **ZWEITEILIGES MEDIZINISCHES INSTRUMENT**
TWO-PART MEDICAL INSTRUMENT
INSTRUMENT MEDICAL EN DEUX PARTIES

(30) Priorität: 05.09.2002 DE 10242152
(43) Veröffentlichungstag der Anmeldung: 01.06.2005
(73) Patentinhaber: Aesculap AG, 78532 Tuttlingen (DE)
(72) Erfinder: DWORSCHAK, Manfred, 78589 Dürbheim (DE); LUTZE, Theodor, 78582 Balgheim (DE); MORALES, Pedro, 78532 Tuttlingen-Nendingen (DE); WEISSHAUPT, Dieter, 78194 Immendingen (DE)
(74) Vertreter: Regelmann, Thomas
(86) Internationale Anmeldenummer: PCT/EP2003/009433
(87) Internationale Veröffentlichungsnummer: WO 2004/021897

(56) Entgegenhaltungen:
- WO-A-97/07726
- DE-C- 4 339 992
- DE-U- 20 113 017
- DE-U- 20 113 020
- US-A- 3 302 648

## Beschreibung

Die Erfindung betrifft ein zweiteiliges medizinisches Instrument, bestehend aus einem ersten Teil mit einem Zapfen und einem zweiten Teil mit einer Zapfenaufnahme, wobei erstes Teil und zweites Teil über eine Zapfen-Zapfenaufnahme-Verbindung miteinander verbunden sind und das erste Teil aus einem Kunststoffmaterial hergestellt ist und der Zapfen integral an dem ersten Teil gebildet ist.

Aus der US 3,302,648 ist eine einteilige Klammer bekannt, welche zwei Griffhebel aufweist, wobei an einem Griffhebel integral ein Zapfenstummel mit einer geviertelten oder dreigabligen Segmentstruktur sitzt. An dem Zapfenstummel sitzt ein Kopf, welcher segmentweise aufgeteilt ist in Fortsetzung der Zapfenstummelsegmente.

Aus der DE 201 13 017 U1 ist ein medizinisches Instrument mit einem ersten Teil und einem zweiten Teil, welche über eine Verbindungsvorrichtung miteinander verbunden sind, bekannt. An dem ersten Teil ist ein Zapfen angeordnet und das zweite Teil weist eine Zapfenausnehmung zur Aufnahme eines Zapfens auf. An dem zweiten Teil ist an der Zapfenausnehmung eine Querausnehmung gebildet. Zur Herstellung eines Formschlusse mit dem Zapfen und dem zweiten Teil ist ein Schieber vorgesehen, welcher in die Querausnehmung schiebbar ist.

Aus der DE 201 13 020 U1 ist ein medizinisches Instrument mit einem ersten Teil und einem zweiten Teil, welche über eine Verbindungsvorrichtung miteinander verbunden sind, bekannt. Das zweite Teil liegt an dem ersten Teil an und es ist ein Deckelelement vorgesehen, welches mit dem ersten Teil verbunden ist und welches auf das zweite Teil so aufgelegt ist, dass die Relativbewegung des zweiten Teils von dem ersten Teil weg über das Deckelelement gesperrt ist.

Aus der DE 43 39 992 C1 ist ein chirurgisches Instrument mit zwei Branchen, die durch eine durch fluchtende Lageröffnungen der beiden Branchen hindurchragende, mindestens aus zwei Teilen bestehende Lagerwelle schwenkbar miteinander verbunden sind, deren Enden einen größeren Außendurchmesser aufweisen als die Lageröffnungen, bekannt. Die zwei Teile der Lagerwelle gehen durch axiales Zusammenschieben eine elastische Rastverbindung ein.

Der Erfindung liegt die Aufgabe zugrunde, ein zweiteiliges medizinisches Instrument der eingangs genannten Art bereitzustellen, welches auf einfache Weise herstellbar ist.

Diese Aufgabe wird bei dem eingangs genannten zweiteiligen medizinischen Instrument erfindungsgemäß dadurch gelöst, daß der Zapfen einen ohne Unterbrechungen umlaufenden Randwulst aufweist, daß der Zapfen mit dem Randwulst in Relation mit der Zapfenaufnahme so dimensioniert und so ausgebildet ist, daß er aufgrund seiner Elastizität in die Zapfenaufnahme schiebbar ist, und daß die Zapfen-Zapfenaufnahme-Verbindung über den Randwulst hergestellt ist, indem der Randwulst auf eine Oberfläche des zweiten Teils aufgelegt ist.

Ein umlaufender Randwulst an dem Zapfen läßt sich integral mit dem Zapfen auf einfache Weise herstellen, ohne daß beispielsweise getrennte Segmente vorgesehen werden müssen. Insbesondere bei medizinischen Instrumenten, bei denen keine oder nur geringe Querkräfte auftreten, wie bei Fassinstrumenten, läßt sich so eine einfache und kostengünstige Herstellbarkeit erreichen.

Da keine Segmente vorgesehen werden müssen, ist damit auch der Zapfen mit der umlaufenden Randwulst stabiler, so daß die Gefahr eines Wegbrechens eines Segmentes nicht besteht. Dies ist besonders wichtig bei einem medizinischen Instrument, da vermieden werden muß, daß, wenn beispielsweise eine zu hohe Kraft ausgeübt wird, Teile des Instrumentes abbrechen, da diese beispielsweise in eine Wunde fallen können oder die Funktionalität des Instrumentes während eines Bearbeitungsvorgangs am menschlichen Körper gestört wird.

Insbesondere ist es dadurch möglich, das erste Teil mit dem Zapfen auf einfache Weise in einem Gießverfahren herzustellen.

Der Zapfen ist mit der Randwulst in Relation zu der Zapfenaufnahme so dimensioniert und so ausgebildet, daß er aufgrund seiner Elastizität in die Zapfenaufnahme schiebbar ist und insbesondere der Randwulst in die Wulstaufnahme bringbar ist bzw. auf eine Oberfläche des zweiten Teils auflegbar ist. Es läßt sich dann auf diese Weise eine lösbare oder unlösbare Zapfen-Zapfenaufnahme-Verbindung herstellen, wobei es auch noch möglich ist, die beiden Teile relativ zueinander zu schwenken. Es läßt sich so eine sichere Verbindung herstellen, wobei die beiden Einzelteile selber auf einfache Weise hergestellt werden können. Zur Herstellung der Fixierung müssen nur relativ geringe Montagekräfte ausgeübt werden.

Insbesondere ist der Randwulst durch eine Wulstaufnahme aufgenommen, so daß der Zapfen keinen oder nur einen geringen Überstand über das zweite Teil aufweist.

Durch den in der Wulstaufnahme sitzenden Randwulst läßt sich die Anlagefläche zwischen Randwulst und Wulstaufnahme, das heißt die Anlagefläche zwischen Zapfen und Zapfenaufnahme, minimieren, so daß insbesondere eine Schwenkbarkeit der beiden Teile relativ zueinander nicht gesperrt und nicht wesentlich behindert ist. Es lassen sich dann medizinische Instrumente herstellen, wie beispielsweise Klemmen und Scheren, bei denen die relative Schwenkbarkeit der beiden Teile relativ zueinander ausgehend von einer geschlossenen Stellung aus nicht wesentlich behindert ist.

Vorteilhafterweise weist die Zapfenaufnahme im Bereich der Wulstaufnahme einen größeren Durchmesser auf als außerhalb der Wulstaufnahme. Dadurch läßt sich eine Anlagefläche bereitstellen, über die eine Wegbewegung des zweiten Teils von dem ersten Teil sperrbar ist.

Es ist günstig, wenn die Höhe der Wulstaufnahme kleiner ist als die Höhe der Zapfenaufnahme außerhalb der Wulstaufnahme. Dadurch läßt sich der Randwulst auf einfache Weise herstellen, da dieser dann nur einen sehr kleinen Anteil an dem Zapfen darstellt.

Insbesondere ist dabei die Wulstaufnahme an einer Oberfläche des zweiten Teils angeordnet, so daß der Aufwand zur Bildung der Wulstaufnahme minimiert ist, da diese beispielsweise von der Oberfläche her einfräsbar ist oder bei entsprechender Formausbildung mittels eines Gießverfahrens herstellbar ist. Insbesondere ist es vorgesehen, daß die Wulstaufnahme versenkt angeordnet ist. Auf diese Weise läßt sich auch erreichen, daß der Zapfen über das zweite Teil in minimierter Höhe hinausragt.

Weiterhin ist es günstig, wenn die Wulstaufnahme einen ringförmigen Querschnitt um die Zapfenaufnahme aufweist. Dadurch läßt es sich gewährleisten, daß der Zapfen in der Zapfenaufnahme drehbar ist, wobei eine minimierte Anlagefläche bereitgestellt ist, die ausreichend ist, um die Wegbewegung des zweiten Teils vom ersten Teil zu sperren, nicht jedoch die Schwenkbewegung der beiden Teile relativ zueinander.

Der Zapfen kann als Vollzapfen ausgebildet sein oder eine zentrale Ausnehmung aufweisen. Dadurch läßt sich eine elastische Komprimierung des Zapfens quer zu seiner Längsrichtung erreichen, die die Einführbarkeit des Zapfens in die Zapfenaufnahme erleichtert. Es läßt sich dadurch ermöglichen, daß durch elastische Komprimierung quer zur Längsrichtung die Einführung erleichtert wird, während zur Herausführung des Randwulstes aus der Wulstaufnahme eine erheblich höhere Kraft mit einer Richtung im wesentlichen parallel zur Längsrichtung überwunden werden muß (in eine Richtung parallel zu den Normalen der Anlageflächen).

Günstigerweise ist die zentrale Ausnehmung zylindrisch ausgebildet, um so eine rotationssysmmetrische Ausbildung zu erhalten.

Günstigerweise weist die zentrale Ausnehmung eine größere Höhe auf als der Zapfen, das heißt die zentrale Ausnehmung erstreckt sich unterhalb der Oberfläche des ersten Teils, welche dem zweiten Teil zugewandt ist. Dadurch läßt sich eine größere elastische Querbeweglichkeit von Zapfenwänden erreichen, um somit wiederum die Einführung des Zapfens in die Zapfenaufnahme zu erleichtern.

Durch die erfindungsgemäße Lösung ist es grundsätzlich möglich, das zweite Teil mit geringerer Elastizität auszubilden. Vorteilhaft ist es jedoch, wenn das zweite Teil ebenfalls aus einem Kunststoffmaterial hergestellt ist, und insbesondere aus dem gleichen Material wie das erste Kunstoffmaterial hergestellt ist, um so kompatible Eigenschaften der beiden Teile zu erhalten.

Vorteilhafterweise ist der Zapfen rotationssymmetrisch ausgebildet, um so beispielsweise eine gute Verschwenkbarkeit der beiden Teile relativ zueinander zu erhalten.

Aus dem gleichen Grund ist es günstig, wenn die Zapfenaufnahme rotationssysmmetrisch ausgebildet ist.

Das erste Teil mit dem integral an ihm gebildeten Zapfen läßt sich auf einfache Weise herstellen, wenn der Zapfen eine durchlaufende zylindrische Oberfläche aufweist. Durchlaufend bezieht sich dabei auf Richtungen senkrecht zur radialen Richtung. Der Zapfen ist damit nicht segmentweise aufgeteilt, so daß er einfacher herstellbar ist und auch das Abbrechen von einzelnen Segmenten verhindert ist.

Ganz besonders günstig ist es, wenn die Zapfenaufnahme dem ersten Teil zugewandt eine Einschubausnehmung für den Zapfen aufweist. In diese Einschubausnehmung läßt sich dann zum Zusammenbau der beiden Teile der Randwulst einführen und dann der Zapfen nach oben drücken, um so schließlich den Randwulst in seine Wulstaufnahme zu bringen.

Es ist dann ebenfalls günstig, wenn der Zapfen an seinem oberen Ende abgeschrägt ausgebildet ist. Wenn dann der Zapfen in seiner Längsrichtung in die Zapfenaufnahme gedrückt wird, dann werden insbesondere in Zusammenwirkung mit der Einschubausnehmung Querkräfte ausgeübt, die Zapfenwände mit der Randwulst in Richtung der Achse des Zapfens drücken. Dadurch kann dann der Randwulst durch den Teil der Zapfenaufnahme geführt werden, welcher außerhalb der Wulstaufnahme liegt.

Insbesondere ist dabei der Zapfen so ausgebildet, daß er in der Zapfenaufnahme drehbar ist. Es lassen sich dann beispielsweise zweiteilige medizinische Klemmen oder andere Fassinstrumente herstellen.

Weiterhin ist es günstig, wenn der Randwulst so ausgebildet ist, daß über diesen erstes Teil und zweites Teil miteinander fixiert sind, das heißt die Wegbewegung des zweiten Teils von dem ersten Teils gesperrt ist.

Insbesondere ist es vorgesehen, daß der Zapfen so ausgebildet ist, daß er quer zu einer Höhenrichtung eine elastische Dehnung zwischen 1 % und 5 % aufweist. Dadurch ist zum einen sichergestellt, daß eine genügend große Anlagefläche durch den Kontakt zwischen Randwulst und Wulstaufnahme bereitgestellt ist, um die beiden Teil miteinander zu fixieren und zum anderen ist sichergestellt, daß der Zapfen in die Zapfenaufnahme bringbar ist.

Weiterhin ist es günstig, wenn der Randwulst in einer Breite von 1 % bis 5 % über den Zapfenbereich insbesondere quer zu einer Zapfen-Längsrichtung hinausragt, welcher in der Zapfenaufnahme außerhalb des Randwulstes sitzt. Dadurch läßt sich eine einfache Herstellbarkeit der Zapfen-Zapfenaufnahme-Verbindung realisieren, wobei eine sichere Fixierung der beiden Teile erreicht ist.

Die nachfolgende Beschreibung einer bevorzugten Ausführungsform dient in Zusammenhang mit der Zeichnung der näheren Erläuterung der Erfindung. Es zeigen:
- Figur 1: eine Draufsicht auf ein Ausführungsbeispiel eines erfindungs- gemäßen medizinischen Instrumentes;
- Figur 2: eine seitliche Schnittdarstellung einer erfindungsgemäßen Zapfen-Zapfenaufnahme-Verbindung und
- Figur 3: eine vergrößerte Darstellung des Bereiches A gemäß Figur 2.

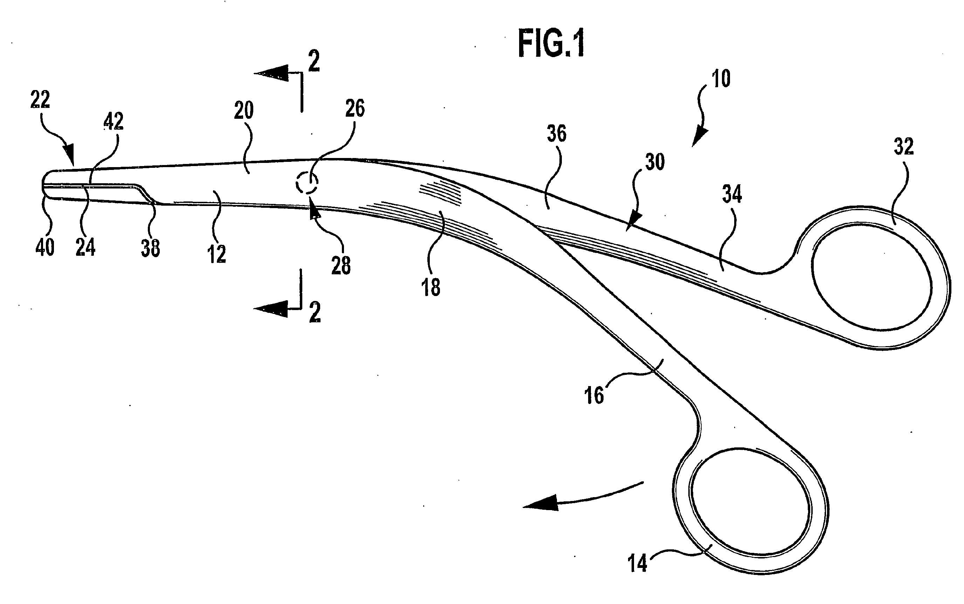

Ein Ausführungsbeispiel eines erfindungsgemäßen zweiteiligen medizinischen Instrumentes ist beispielsweise eine Klemme als Fassinstrument, welche in Figur 1 dargestellt und als Ganzes mit 10 bezeichnet ist. Diese umfaßt ein erstes Teil 12 (männliches Teil), welches aus einem Kunststoffmaterial wie PEEK hergestellt ist. Bei dem gezeigten Ausführungsbeispiel weist das erste Teil 12 eine Grifföse 14 auf, welche an einem Griffösenbereich 16 sitzt. An diesen Griffösenbereich 16 schließt sich ein gebogener Mittelbereich 18 an, welcher in einen Endbereich 20 übergeht. Der Endbereich 20 weist ein Arbeitsende 22 auf, an welchem beispielsweise Griffflächen 24 oder eine Schneidkante gebildet bzw. angeordnet ist.

In dem Mittelbereich 18 sitzt ein Zapfen 26 zur Bildung einer Zapfen-Zapfenaufnahme-Verbindung 28, mittels welcher das erste Teil 12 schwenkbar mit einem zweiten Teil 30 (weibliches Teil) verbunden ist.

Das zweite Teil 30 weist ebenfalls eine Grifföse 32 auf, welche an einem Griffösenbereich 34 sitzt, der in einen gebogenen Mittelbereich 36 übergeht. Bei dem gezeigten Ausführungsbeispiel ist der Mittelbereich 36 des zweiten Teils 30 weniger stark gebogen als der Mittelbereich 18 des ersten Teils 12, so daß bei geschlossener Klemme die beiden Griffösen 32 und 14 beabstandet zu einander sind.

Der Mittelbereich 36 des zweiten Teils 30 geht in einen Endbereich 38 über, welcher ein Arbeitsende 40 beispielsweise mit Griffflächen 42 aufweist, welche den Griffflächen 24 des ersten Teils 12 zugewandt sind, so daß zwischen ihnen ein Gegenstand gehalten werden kann.

Die Endbereiche 20 und 38 des ersten Teils 12 und des zweiten Teils 30 sind dabei so ausgestaltet und auf einander abgestimmt, daß die Schwenkbarkeit der beiden Teile 12 und 30 aufeinanderzu gesperrt ist, wenn die geschlossene Stellung erreicht ist, in der beispielsweise die Griffflächen 24 und 42 zumindest teilweise aneinander anliegen.

Aus dieser geschlossenen Stellung heraus läßt sich das erste Teil 12 derart von dem zweiten Teil 30 wegschwenken, daß sich der Abstand zwischen den Griffflächen 24 und 42 vergrößert, das heißt sich ein Maul öffnet. Durch Heranschwenken des ersten Teils 12 auf das zweite Teil 30 zu und Ausüben einer Kraft läßt sich ein Gegenstand zwischen den Griffflächen 24 und 42 klemmen, wobei insbesondere durch die gebogene Ausbildung des ersten Teils 12 verstärkt eine entsprechende Hebelkraft auf den Gegenstand ausübbar ist.

Das erste Teil 12 weist dem zweiten Teil 30 zugewandt eine ebene Oberfläche 44 auf. Das zweite Teil 30 weist dem ersten Teil 12 zugewandt ebenfalls eine ebene Oberfläche 46 auf. Der Zapfen 26 steht über diese ebene Oberfläche 44 des ersten Teils 12 hinaus, wobei der Zapfen 26 einstückig mit dem ersten Teil 12 verbunden ist.

Der Zapfen 26 umfaßt einen ersten zylindrischen Bereich 48, welcher eine äußere Oberfläche aufweist, die einem Mantel eines Zylinders entspricht und rotationssymmetrisch zu einer Längsachse 50 des Zapfens 26 ist. Der Zapfen 26 weist im ersten zylindrischen Bereich 48 eine im wesentlichen glatte Oberfläche ohne Unterbrechungen, Ausnehmungen und dergleichen auf.

An den ersten zylindrischen Bereich 48 schließt sich ein Randwulst 52 an, die in der Art eines Ringes umlaufend ist, wobei im Bereich der Randwulst 52 der Zapfen 26 einen größeren Durchmesser aufweist als am ersten zylindrischen Bereich 48. Der Randwulst 52 ragt dabei über den ersten zylindrischen Bereich 48 quer zur Längsachse 50 in einer radialen Breite d hinaus, welche im Bereich zwischen ca. 1 % bis 5 % des Radius R des Zapfens 26 im ersten zylindrischen Bereich 48 liegt. Die Übergänge zwischen den Bereichen und Kanten sind vorzugsweise abgerundet ausgebildet.

Die Höhe des ersten zylindrischen Bereichs 48 parallel zur Längsachse 50 ist erheblich höher als die Höhe des Randwulstes 52 in dieser Richtung.

An den Randwulst 52 schließt sich in Richtung der Längsachse 50 ein abgefaster Bereich 54 an, welcher kegelstumpfförmig ist. Er weist somit eine ringförmig umlaufende abgeschrägte Fläche 56 auf, welche wie unten noch näher erläutert zur leichteren Einführung des Zapfens 26 in eine Zapfenaufnahme 58 des zweiten Teils 30 dient.

Der Zapfen 26 weist eine zentrale Ausnehmung 60 auf, welche zylindrisch ausgebildet ist und insbesondere konzentrisch um die Längsachse 50 des Zapfens 26 ist. Die Höhe dieser zentralen Ausnehmung 60 von einem oberen Ende 62 des Zapfens 26 aus gemessen ist dabei derart, daß sich die zentrale Ausnehmung 60 über die Oberfläche 44 des ersten Teils 12 hinaus erstreckt, das heißt ein dem oberen Ende 62 des Zapfens 26 abgewandtes unteres Ende 64 der zentralen Ausnehmung 60, welche den Boden der zentralen Ausnehmung 60 bildet, liegt unterhalb der Oberfläche 44 des ersten Teils 12.

Der Zapfen 26 ist so dimensioniert und so ausgebildet, daß in Zusammenwirkung mit der zentralen Ausnehmung 60 eine elastische Kompressibilität quer zur Längsachse 50 im Bereich zwischen ca. 1 % und 5 % erreichbar ist, um die Einführbarkeit des Zapfens 26 in die Zapfenausnahme 58 zu ermöglichen und um über den Randwulst 52 eine Zapfen-Zapfenaufnahme-Verbindung herzustellen, über welche das erste Teil 12 und das zweite Teil 30 drehbar aneinander gehalten sind mit einer Drehachse, welche mit der Längsachse 50 zusammenfällt.

Insbesondere ist das erste Teil 12 aus einem derartigen Material und insbesondere Kunststoffmaterial hergestellt, welches eine elastische Dehnung im Bereich zwischen ca. 1 % und 5 % aufweist.

Die in dem zweiten Teil 30 gebildete Zapfenaufnahme 58 dient zur Aufnahme des Zapfens 26. Die Zapfenaufnahme 58 ist durch eine zylindrische Öffnung in dem zweiten Teil 30 gebildet, welche koaxial mit der Längsachse 50 verläuft.

Das zweite Teil 30 ist an der Zapfenaufnahme 58 dem ersten Teil 12 zugewandt abgeschrägt mit einer umlaufenden schrägen Fläche 66 ausgebildet. Die schräge Fläche 66, die eine Mantelfläche eines Kegelstumpfes ist, ist dabei so abgestimmt auf die abgeschrägte Fläche 56 des Zapfens 26, daß dessen Einführung in das zweite Teil 30 von Richtung der Oberfläche 46 her erleichtert ist; die schräge Fläche 66 bildet damit an der Zapfenaufnahme 58 eine Einführausnehmung 68 für den Zapfen 26 aus.

Die Zapfenaufnahme 58 umfaßt ferner eine Wulstaufnahme 70 zur Aufnahme des Randwulstes 52 des Zapfens 26. Diese Wulstaufnahme 70 ist als im Querschnitt ringförmige Ausnehmung 72 an einer Oberfläche 74 des zweiten Teils 30 gebildet, wobei diese Oberfläche 74 der anderen Oberfläche 46 gegenüber liegt.

Die Wulstaufnahme 70 weist in der Richtung der Längsachse 50 eine kleinere Höhe auf als die restliche Zapfenaufnahme 58 außerhalb der Wulstaufnahme 70. Die Wulstaufnahme 70 ist gegenüber der Oberfläche 74 zurückgesetzt, das heißt gegenüber dieser versenkt ausgebildet, so daß der Randwulst 52 zumindest teilweise unterhalb der Oberfläche 74 liegend in die Wulstaufnahme 70 eingetaucht ist. Übergänge und Kanten an der Wulstaufnahme 70 sind vorzugsweise abgerundet ausgebildet.

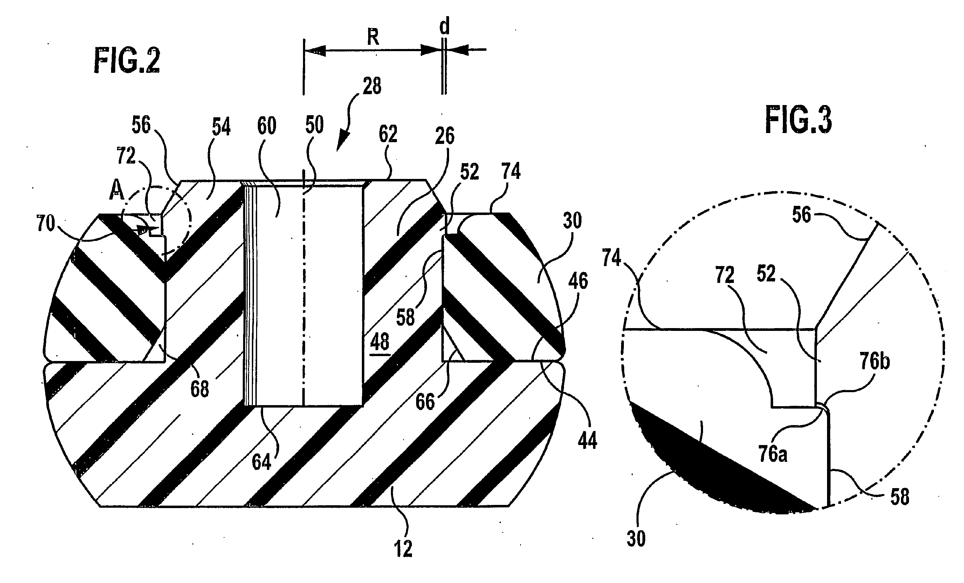

Bei einer bevorzugten Variante einer Ausführungsform, welche in Figur 2 gezeigt ist, verläuft der Randwulst fluchtend mit der Oberfläche 74 des zweiten Teils 30, so daß nur der Bereich 54 des Zapfens 26 über das zweite Teil 30 hinausragt, nicht jedoch der Randwulst 52.

An dem Randwulst 52 und der Wulstaufnahme 70 sind jeweils ringförmige Anlageflächen 76a und 76b gebildet, mittels welchen die Wegbewegung des zweiten Teils 30 von dem ersten Teil 12 gesperrt ist. Die Zubewegung des zweiten Teils 30 auf das erste Teil 12 ist durch Anlage der Oberflächen 44 und 46 gesperrt. Die Anlageflächen 76a, 76b sind dabei so dimensioniert, daß bei der Wegbewegung des zweiten Teils 30 von dem ersten Teil 12 die elastische Kraft des Zapfens 26 am Randwulst 52 im wesentlichen parallel zur Längsachse 50 überwinden werden muß. Diese zur überwindende elastische Kraft ist dabei erheblich höher als die elastische Querkraft, die überwunden werden muß, wenn der Zapfen 26 in die Zapfenaufnahme 58 eingeführt wird.

Insbesondere liegt im Bereich der Anlagefläche 76b der Zapfenaufnahme 58 ein in Querschnitt stufenförmiger Übergang vor, das heißt die Anlagefläche 76b liegt im wesentlichen senkrecht zu einer Hohlzylindermantelfläche der Zapfenaufnahme 58 in dem Bereich, in dem durch die Zapfenaufnahme 58 der erste zylindrische Bereich 48 des Zapfens 26 aufgenommen ist.

Das zweite Teil 30 kann ebenfalls aus einem Kunststoffmaterial hergestellt sein. Insbesondere ist es aus dem gleichen Material hergestellt wie das erste Teil 12.

Die beiden Teile 12 und 30 werden getrennt hergestellt, wobei es sich jeweils um einstückige Teile handelt. (Arbeitswerkzeuge wie Schneiden können getrennt ausgebildet sein.) Es wird dabei in dem ersten Teil 12 der Zapfen 26 gebildet und in dem zweiten Teil die Zapfenaufnahme 58.

Der Zapfen 26 wird über die Einführausnehmung 68 in die Zapfenaufnahme 58 geschoben (bzw. umgekehrt die Zapfenaufnahme 58 auf den Zapfen 26 aufgeschoben).

Aufgrund der elastischen Eigenschaften des Materials des ersten Teils 12 und der zentralen Ausnehmung 60 kann dann der Randwulst 52 in der Zapfenaufnahme 58 in Richtung der Längsachse 50 verschoben werden, bis die Wulstaufnahme 70 erreicht ist und dort der Randwulst 52 quer zur Längsachse 50 zurückfedert und damit durch die Wulstaufnahme 70 aufgenommen ist. Dadurch ist dann das zweite Teil 30 drehbar an dem ersten Teil 12 fixiert, wobei sich die Einzelteile 12 und 30 mit geringen Montagekräften fügen lassen. Die Montage kann dabei von Hand erfolgen oder maschinell erfolgen.

Durch entsprechende Materialwahl insbesondere für das erste Teil 12 mit dem Zapfen 26 mit einem Kunststoffwerkstoff, welcher eine elastische Dehnung im

Bereich zwischen ca. 1 % bis 5 % aufweist, läßt sich so eine sichere Verbindung als Zapfenverriegelung zwischen den beiden Teilen 12 und 30 herstellen.

Bei entsprechender Dimension des Randwulstes 52, der umlaufend ist ohne Unterbrechungen, läßt sich eine lösbare oder eine unlösbare Zapfenverriegelung herstellen.

## Patentansprüche

1. Zweiteiliges medizinisches Instrument, bestehend aus einem ersten Teil (12) mit einem Zapfen (26) und einem zweiten Teil (30) mit einer Zapfenaufnahme (58), wobei erstes Teil (12) und zweites Teil (30) über eine Zapfen-Zapfenaufnahme-Verbindung (28) miteinander verbunden sind, das erste Teil (12) aus einem Kunststoffmaterial hergestellt ist, und der Zapfen (26) integral an dem ersten Teil (12) gebildet ist, wobei der Zapfen (26) einen ohne Unterbrechungen umlaufenden Randwulst (52) aufweist, der Zapfen (26) mit dem Randwulst (52) in Relation mit der Zapfenaufnahme (58) so dimensioniert und so ausgebildet ist, daß er aufgrund seiner Elastizität in die Zapfenaufnahme (58) schiebbar ist, und die Zapfen-Zapfenaufnahme-Verbindung (28) über den Randwulst (52) hergestellt ist, indem der Randwulst (52) auf eine Oberfläche des zweiten Teils (30) aufgelegt ist.

2. Zweiteiliges medizinisches Instrument nach Anspruch 1, **dadurch gekennzeichnet, daß** die Zapfenaufnahme (58) eine Wulstaufnahme (70) für den Randwulst (52) umfaßt.

3. Zweiteiliges medizinisches Instrument nach Anspruch 2, **dadurch gekennzeichnet, daß** die Zapfenaufnahme (58) im Bereich der Wulstaufnahme (70) einen größeren Durchmesser aufweist als außerhalb der Wulstaufnahme (70).

4. Zweiteiliges medizinisches Instrument nach Anspruch 2 oder 3, **dadurch gekennzeichnet, daß** die Höhe der Wulstaufnahme (70) in einer Längsrichtung (50) des Zapfens (26) kleiner ist als die Höhe der Zapfenaufnahme (58) außerhalb der Wulstaufnahme (70).

5. Zweiteiliges medizinisches Instrument nach einem der Ansprüche 2 bis 4, **dadurch gekennzeichnet, daß** die Wulstaufnahme (70) an einer Oberfläche (74) des zweiten Teils (30) angeordnet ist.

6. Zweiteiliges medizinisches Instrument nach Anspruch 5, **dadurch gekennzeichnet, daß** die Wulstaufnahme (70) versenkt angeordnet ist.

7. Zweiteiliges medizinisches Instrument nach einem der Ansprüche 2 bis 6, **dadurch gekennzeichnet, daß** die Wulstaufnahme (70) einen ringförmigen Querschnitt um die Zapfenaufnahme (58) aufweist.

8. Zweiteiliges medizinisches Instrument nach einem der vorangehenden Ansprüche, **dadurch gekennzeichnet, daß** der Zapfen (26) eine zentrale Ausnehmung (60) aufweist.

9. Zweiteiliges medizinisches Instrument nach Anspruch 8, **dadurch gekennzeichnet, daß** die zentrale Ausnehmung (60) zylindrisch ausgebildet ist.

10. Zweiteiliges medizinisches Instrument nach Anspruch 8 oder 9, **dadurch gekennzeichnet, daß** die zentrale Ausnehmung (60) eine größere Höhe aufweist als der Zapfen (26).

11. Zweiteiliges medizinisches Instrument nach einem der Ansprüche 8 bis 10, **dadurch gekennzeichnet, daß** die zentrale Ausnehmung (60) sich unterhalb einer Oberfläche (44) des ersten Teils (12) erstreckt.

12. Zweiteiliges medizinisches Instrument nach einem der vorangehenden Ansprüche, **dadurch gekennzeichnet, daß** das zweite Teil (30) aus einem Kunststoffmaterial hergestellt ist.

13. Zweiteiliges medizinisches Instrument nach einem der vorangehenden Ansprüche, **dadurch gekennzeichnet, daß** der Zapfen (26) rotationssysmmetrisch ausgebildet ist.

14. Zweiteiliges medizinisches Instrument nach Anspruch 13, **dadurch gekennzeichnet, daß** der Zapfen (26) eine durchlaufende zylindrische Oberfläche aufweist.

15. Zweiteiliges medizinisches Instrument nach einem der vorangehenden Ansprüche, **dadurch gekennzeichnet, daß** die Zapfenaufnahme (58) rotationssysmmetrisch ausgebildet ist.

16. Zweiteiliges medizinisches Instrument nach einem der vorangehenden Ansprüche, **dadurch gekennzeichnet, daß** die Zapfenaufnahme (58) dem ersten Teil (12) zugewandt eine Einführausnehmung (68) für den Zapfen (26) aufweist.

17. Zweiteiliges medizinisches Instrument nach einem der vorangehenden Ansprüche, **dadurch gekennzeichnet, daß** der Zapfen (26) an seinem oberen Ende abgeschrägt ausgebildet ist.

18. Zweiteiliges medizinisches Instrument nach einem der vorangehenden Ansprüche, **dadurch gekennzeichnet, daß** der Zapfen (26) und der Randwulst (52) so ausgebildet sind, daß der Zapfen (26) mit dem Randwulst (52) in die Wulstaufnahme (70) bringbar ist.

19. Zweiteiliges medizinisches Instrument nach einem der vorangehenden Ansprüche, **dadurch gekennzeichnet, daß** der Zapfen (26) so ausgebildet ist, daß er in der Zapfenaufnahme (58) drehbar ist.

20. Zweiteiliges medizinisches Instrument nach einem der vorangehenden Ansprüche, **dadurch gekennzeichnet, daß** der Randwulst (52) so ausgebildet ist, daß über diese erstes Teil (12) und zweites Teil (30) miteinander fixiert sind.

21. Zweiteiliges medizinisches Instrument nach einem der vorangehenden Ansprüche, **dadurch gekennzeichnet, daß** der Zapfen (26) so ausgebildet ist, daß er quer zu einer Höhenrichtung (50) eine elastische Dehnung zwischen 1 % und 5 % aufweist.

22. Zweiteiliges medizinisches Instrument nach einem der vorangehenden Ansprüche, **dadurch gekennzeichnet, daß** der Randwulst (52) in einer Richtung in einer Breite (d) von 1 % bis 5 % über den Zapfenbereich (48) außerhalb des Randwulstes (52) hinausragt.

23. Zweiteiliges medizinisches Instrument nach einem der vorangehenden Ansprüche, **dadurch gekennzeichnet, daß** der Randwulst (52) quer zu einer Längsrichtung (50) des Zapfens (26) über einen Bereich außerhalb des Randwulstes (52) hinausragt.

## Claims

1. Two-part medical instrument, consisting of a first part (12) having a peg (26) and a second part (30) having a peg receiver (58), wherein the first part (12) and the second part (30) are connected to one another by a peg/peg receiver connection (28), wherein the first part (12) is made of a plastics material and the peg (26) is integrally formed on the first part (12), wherein the peg (26) has an edge bead (52) surrounding said peg without interruptions, wherein the peg (26) having the edge bead (52) is so dimensioned and designed in relation to the peg receiver (58) that the peg owing to its elasticity is pushable into the peg receiver (58), and wherein the peg/peg receiver connection (28) is established via the edge bead (52) by the edge bead (52) being placed on a surface of the second part (30).

2. Two-part medical instrument according to claim 1, **characterized in that** the peg receiver (58) comprises a bead receiver (70) for the edge bead (52).

3. Two-part medical instrument according to claim 2, **characterized in that** the peg receiver (58) has a larger diameter in the region of the bead receiver (70) than outside of the bead receiver (70).

4. Two-part medical instrument according to claim 2 or 3, **characterized in that** the height of the bead receiver (70) in a longitudinal direction (50) of the peg (26) is smaller than the height of the peg receiver (58) outside of the bead receiver (70).

5. Two-part medical instrument according to any one of claims 2 to 4, **characterized in that** the bead receiver (70) is disposed at a surface (74) of the second part (30).

6. Two-part medical instrument according to claim 5, **characterized in that** the bead receiver (70) is disposed in a countersunk manner.

7. Two-part medical instrument according to any one of claims 2 to 6, **characterized in that** the bead receiver (70) has an annular cross section around the peg receiver (58).

8. Two-part medical instrument according to any one of the preceding claims, **characterized in that** the peg (26) has a central recess (60).

9. Two-part medical instrument according to claim 8, **characterized in that** the central recess (60) is of a cylindrical shape.

10. Two-part medical instrument according to claim 8 or 9, **characterized in that** the central recess (60) has a greater height than the peg (26).

11. Two-part medical instrument according to any one of claims 8 to 10, **characterized in that** the central recess (60) extends below a surface (44) of the first part (12).

12. Two-part medical instrument according to any one of the preceding claims, **characterized in that** the second part (30) is made of a plastics material.

13. Two-part medical instrument according to any one of the preceding claims, **characterized in that** the peg (26) is of a rotationally symmetrical shape.

14. Two-part medical instrument according to claim 13, **characterized in that** the peg (26) has a continuous cylindrical surface.

15. Two-part medical instrument according to any one of the preceding claims, **characterized in that** the peg receiver (58) is of a rotationally symmetrical shape.

16. Two-part medical instrument according to any one of the preceding claims, **characterized in that** the peg receiver (58) has an insertion recess (68) for the peg (26) that faces the first part (12).

17. Two-part medical instrument according to any one of the preceding claims, **characterized in that** the peg (26) is bevelled at its top end.

18. Two-part medical instrument according to any one of the preceding claims, **characterized in that** the peg (26) and the edge bead (52) are so designed that the peg (26) is bringable with the edge bead (52) into the bead receiver (70).

19. Two-part medical instrument according to any one of the preceding claims, **characterized in that** the peg (26) is so designed that it is rotatable in the peg receiver (58).

20. Two-part medical instrument according to any one of the preceding claims, **characterized in that** the edge bead (52) is so designed that first part (12) and second part (30) are fastened to one another by said edge bead.

21. Two-part medical instrument according to any one of the preceding claims, **characterized in that** the peg (26) is so designed that it has an elastic extensibility of between 1% and 5% transversely of a height direction (50).

22. Two-part medical instrument according to any one of the preceding claims, **characterized in that** the edge bead (52) projects in one direction by a width (d) of 1% to 5% beyond the peg region (48) outside of the edge bead (52).

23. Two-part medical instrument according to any one of the preceding claims, **characterized in that** the edge bead (52) projects transversely of a longitudinal direction (50) of the peg (26) beyond a region outside of the edge bead (52).

## Revendications

1. Instrument médical en deux parties constitué d'une première partie (12) avec une cheville (26) et d'une seconde partie (30) avec un logement de cheville (58), la première partie (12) et la seconde partie (30) étant raccordées l'une à l'autre au moyen d'une liaison cheville-logement de cheville (28), la première partie (12) étant fabriquée en matière plastique et la cheville (26) étant conçue d'un seul tenant sur la première partie (12), la cheville (26) présentant un rebord périphérique (52) continu, la cheville (26) avec le rebord (52) en relation avec le logement de cheville (58) étant dimensionnée et réalisée de manière à pouvoir être insérée dans le logement de cheville (58) en raison de son élasticité et la liaison cheville-logement de cheville (28) est créée au moyen du rebord (52) en positionnant le rebord (52) sur une surface de la seconde partie (30).

2. Instrument médical en deux parties selon la revendication 1, **caractérisé en ce que** le logement de cheville (58) comprend un logement de rebord (70) pour le rebord (52).

3. Instrument médical en deux parties selon la revendication 2, **caractérisé en ce que** le logement de cheville (58) présente, dans la zone du logement de rebord (70), un diamètre plus important qu'à l'extérieur du logement de rebord (70).

4. Instrument médical en deux parties selon la revendication 2 ou 3, **caractérisé en ce que** le logement de rebord (70) dans un sens longitudinal (50) de la cheville (26) est moins haut que le logement de cheville (58) à l'extérieur du logement de rebord (70).

5. Instrument médical en deux parties selon l'une des revendications 2 à 4, **caractérisé en ce que** le logement de rebord (70) est agencé sur une surface (74) de la seconde partie (30).

6. Instrument médical en deux parties selon la revendication 5, **caractérisé en ce que** le logement de rebord (70) est agencé de manière à être enfoncé.

7. Instrument médical en deux parties selon l'une des revendications 2 à 6, **caractérisé en ce que** le logement de rebord (70) présente une coupe transversale circulaire autour du logement de cheville (58).

8. Instrument médical en deux parties selon l'une quelconque des revendications précédentes, **caractérisé en ce que** la cheville (26) présente un évidement central (60).

9. Instrument médical en deux parties selon la revendication 8, **caractérisé en ce que** l'évidement central (60) est de forme cylindrique.

10. Instrument médical en deux parties selon la revendication 8 ou 9, **caractérisé en ce que** l'évidement central (60) est plus haut que la cheville (26) .

11. Instrument médical en deux parties selon l'une des revendications 8 à 10, **caractérisé en ce que** l'évidement central (60) s'étend sous une surface (44) de la première partie (12).

12. Instrument médical en deux parties selon l'une quelconque des revendications précédentes, **caractérisé en ce que** la seconde partie (30) est fabriquée en matière plastique.

13. Instrument médical en deux parties selon l'une quelconque des revendications précédentes, **caractérisé en ce que** la cheville (26) est conçue de manière à présenter une symétrie de rotation.

14. Instrument médical en deux parties selon la revendication 13, **caractérisé en ce que** la cheville (26) présente une surface cylindrique continue.

15. Instrument médical en deux parties selon l'une quelconque des revendications précédentes, **caractérisé en ce que** le logement de cheville (58) est conçu de manière à présenter une symétrie de rotation.

16. Instrument médical en deux parties selon l'une quelconque des revendications précédentes, **caractérisé en ce que** le logement de cheville (58) en face de la première partie (12) présente un évidement d'insertion (68) pour la cheville (26).

17. Instrument médical en deux parties selon l'une quelconque des revendications précédentes, **caractérisé en ce que** la cheville (26) est biseautée sur son extrémité supérieure.

18. Instrument médical en deux parties selon l'une quelconque des revendications précédentes, **caractérisé en ce que** la cheville (26) et le rebord (52) sont conçus de manière à ce que la cheville (26) avec le rebord (52) puisse être amenée dans le logement de rebord (70).

19. Instrument médical en deux parties selon l'une quelconque des revendications précédentes, **caractérisé en ce que** la cheville (26) est conçue de manière à pouvoir pivoter dans le logement de cheville (58).

20. Instrument médical en deux parties selon l'une quelconque des revendications précédentes, **caractérisé en ce que** le rebord (52) est conçu de manière à fixer l'une à l'autre la première partie (12) et la seconde partie (30).

21. Instrument médical en deux parties selon l'une quelconque des revendications précédentes, **caractérisé en ce que** la cheville (26) est conçue de manière à présenter un allongement élastique compris entre 1 % et 5 % transversalement à une direction verticale (50).

22. Instrument médical en deux parties selon l'une quelconque des revendications précédentes, **caractérisé en ce que** le rebord (52) dépasse, dans un sens sur une largeur (d) de 1 % à 5 %, sur la zone de la cheville (48) à l'extérieur du rebord (52).

23. Instrument médical en deux parties selon l'une quelconque des revendications précédentes, **caractérisé en ce que** le rebord (52) dépasse, transversalement à un sens longitudinal (50) de la cheville (26), sur une zone à l'extérieur du rebord (52).
